# EUROPEAN PATENT SPECIFICATION

(11) **EP 2 512 602 B1**
(45) Date of publication and mention of the grant of the patent: **22.11.2017**
(21) Application number: 09801299.0
(22) Date of filing: 18.12.2009
(51) Int. Cl.: A61Q 5/00, A61Q 5/04, A61K 8/81, A61Q 5/06, A61K 8/73, A61K 8/84, A61K 8/88, A45D 7/02

(54) **PROCESS FOR TREATING KERATIN FIBERS**
VERFAHREN ZUR BEHANDLUNG VON KERATINFASERN
PROCÉDÉ DE TRAITEMENT DE FIBRES DE KÉRATINE

(43) Date of publication of application: 24.10.2012
(73) Proprietor: L'Oréal, 75008 Paris (FR)
(72) Inventor: DE BONI, Maxime, Kawasaki-shi Kanagawa 213-0012 (JP); TAKAHASHI, Hiroshi, Kawasaki-shi Kanagawa 213-0012 (JP)
(74) Representative: Lavoix
(86) International application number: PCT/JP2009/071731
(87) International publication number: WO 2011/074140

(56) References cited:
- EP-A2- 2 111 852
- JP-A- 2007 039 410
- US-A- 4 658 839
- US-A1- 2002 157 682
- US-A1- 2009 048 132

## Description

### TECHNICAL FIELD

The present invention relates to a process for treating keratin fibers such as hair.

### BACKGROUND ART

Due to the many physical stresses (UV, shampoo, brushing, and the like) and chemical stresses (coloration, perm, relaxing, pollution, and the like) that keratin fibers such as hair must undergo daily, research for effectively repairing damaged keratin fibers has become important in the cosmetic treatments for keratin fibers.

Repairing damaged keratin fibers is only worthwhile if a real sensation of return to the original state of the keratin fibers is perceived. Furthermore, the repairing treatment should be effective against various stresses, as mentioned before.

Technologies (compositions and/or processes) known as treatments for repairing damaged keratin fibers that have been proposed are still insufficient insofar as they are often temporary and do not achieve proper recovery of keratin fiber integrity.

Cationic polymers are widely used in the formulation of hair care products. For example, please refer to US-A-2007-105734.

JP 2007-039410 discloses a hair treatment process comprising the steps of: pre-treating hair with an acid; conditioning the hair with Polyquaternium-11 and a hydrogenated castor oil/sebacic acid copolymer; winding hair around a rod; heating the rod; and post-treating the hair with shampooing and rinsing, wherein the heating step is not performed in an occlusive condition, and wherein the hair is completely dried by heating step (d).

### DISCLOSURE OF INVENTION

Cationic polymers are expected to improve the cosmetic properties of keratin fibers, such as hair, in particular damaged hair, because they have good affinity due to the cationic moieties with anionic groups on the keratin fibers.

Nevertheless, their benefits on keratin fibers are still insufficient due to their low deposition yield onto the keratin fibers and low penetration into the keratin fibers. This means that the efficiency of the cosmetic treatment of keratin fibers using a cationic polymer is low and a large quantity of cationic polymer is necessary in the formulation used for the cosmetic treatment.

Thus, an objective of the present invention is to provide a new treatment process for keratin fibers such as hair, using a cationic polymer, even with a relatively small amount thereof, which provides the keratin fibers with good cosmetic effects, in particular superior repairing or recovering effects, which can be effective against various stresses for a long time.

The above objective of the present invention can be achieved by a process for treating keratin fibers comprising the steps of:
applying onto the keratin fibers a composition comprising at least one cationic polymer;
then placing the keratin fibers in an occlusive space to restrict the evaporation of evaporable components in the composition and keep the keratin fibers wet; and then heating the keratin fibers,
wherein
the composition contains neither a reducing agent nor a source of carbonate ions of the formula: 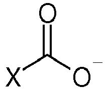
wherein
X is a group selected from the group consisting of O⁻, OH, NH₂, O-OH, and O-COO⁻.

The above process may further comprise the step of rinsing the keratin fibers after the step of applying the composition onto the keratin fibers and/or after the step of heating the keratin fibers.

Mechanical tension may be provided to the keratin fibers. The mechanical tension may be provided by using at least one reshaping means selected from the group consisting of a curler, a roller, a plate and an iron.

The occlusive space may be formed by at least one coating means. The coating means may be rigid or flexible. The coating means may comprise at least one member selected from the group consisting of a film and a sheet.

In the above process, the keratin fibers are heated at 45ºC to 250ºC during the step of heating the keratin fibers while keeping the keratin fibers in the wet state. The keratin fibers may be heated by at least one heater providing at least one selected from the group consisting of hot air, hot steam, high frequency induction heating, microwave heating, infrared ray irradiation, laser, and flash lamp irradiation. The coating means and/or the reshaping means may comprise the heater.

The cationic polymer may have a molecular weight of more than 5000.

The cationic polymer may have no siloxane moiety.

The cationic polymer may comprise at least one group chosen from primary, secondary, tertiary, and/or quaternary amine groups which may either be part of the main polymer chain, or may be carried by a side substituent directly connected to the main chain.

The cationic polymer may be selected from the group consisting of
(1) homopolymers and copolymers derived from acrylic or methacrylic esters and amides;
(2) cationic polysaccharides;
(3) polyaminoamides and derivatives thereof;
(4) cyclopolymers of alkyldiallylamine or dialkyldiallylammonium;
(5) quaternary diammonium polymers;
(6) quaternary polyammonium polymers; and
(7) quaternary polymers of vinylpyrrolidone or vinylimidazole.

The composition may comprise the cationic polymer in an amount of 0.01 to 10% by weight relative to the total weight of the composition. The pH of the composition may range from 6 to 11.

The present application also discloses a composition for treating keratin fibers to be heated in an occlusive space, comprising at least one cationic polymer,
wherein
the composition contains neither a reducing agent nor a source of carbonate ions of the formula: 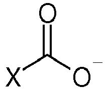 wherein
X is a group selected from the group consisting of O⁻, OH, NH₂, O-OH, and O-COO⁻.

The present application also discloses a kit for treating keratin fibers, comprising:
a device comprising
at least one coating means to form an occlusive space, and
at least one heater to heat the keratin fibers in the occlusive space;
   and
a composition comprising at least one cationic polymer,
wherein
the composition contains neither a reducing agent nor a source of carbonate ions of the formula: 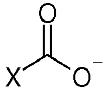
wherein
X is a group selected from the group consisting of O⁻, OH, NH₂, O-OH, and O-COO⁻.

### BEST MODE FOR CARRYING OUT OF THE INVENTION

After diligent research, the inventors have discovered that it is possible to provide keratin fibers such as hair with good cosmetic effects, by using a cationic polymer, and in particular superior repairing or recovering effects, which can be effective against various stresses for a long time, by using a composition comprising a cationic polymer with a specific heating process for the keratin fibers.

The above specific heating process is performed in a closed or occlusive environment, which limits the evaporation of water or moisture from the keratin fibers and maintains the keratin fibers at higher temperature in the wet state. Accordingly, the keratin fibers can be evenly heated, and the cationic polymer can easily penetrate into or deposit onto the keratin fibers such that it can remain in or on the keratin fibers for a long time even after some stresses such as shampooing.

Since a cationic polymer can easily stay on or in the keratin fibers, the process according to the present invention can exhibit good cosmetic effects by using even a relatively small amount of cationic polymer as compared to a conventional process in which it is difficult for a cationic polymer to stay on or in the keratin fibers.

The composition used in the present invention must not contain any reducing agents such as thiol-compounds. Therefore, malodor derived from the reducing agents can be prevented. Furthermore, the composition used in the present invention must not contain any carbonate ion source as defined above. Therefore, cosmetic treatment is more effective, because there is no possibility of producing carbon dioxide which may form a foam that may inhibit the deposition or penetration of the cationic polymer on or into the keratin fibers.

### (Composition)

The composition used for the present invention comprises at least one cationic polymer.

As used herein, the term "cationic polymer" is intended to mean any polymer containing at least one cationic group and/or at least one group which can be ionized to a cationic group. The term "polymer" here means a molecule having repeating units which preferably have a molecular weight of more than 5000, in particular 10000.

The cationic polymer to be used for the present invention may or may not have a siloxane moiety.

The cationic polymers which can be used in accordance with the present invention may be chosen from all those known in the art to improve the cosmetic properties of hair treated with detergent compositions, for example, those described in European Patent Application No. 0 337 354 and French Patent Application Nos. 2 270 846, 2 383 660, 2 598 611, 2 470 596, and 2 519 863.

In one embodiment, the cationic polymers are chosen, for example, from those which comprise units comprising at least one group chosen from primary, secondary, tertiary, and quaternary amine groups which can either be part of the main polymer chain, or can be carried by a side substituent directly connected to the chain.

The cationic polymers may have a weight-average molecular mass of greater than 10⁵, for example, greater than 10⁶, or ranging from 10⁶ to 10⁸.

According to another embodiment, the cationic polymers may be chosen, for instance, from polyamine, polyaminoamide, and polyquaternary ammonium polymers, such as those described in French Patent Nos 2 505 348 and 2 542 997. Non-limiting examples of these polymers include:
(1) homopolymers and copolymers derived from acrylic or methacrylic esters and amides and comprising at least one unit chosen from units of the following formulas: 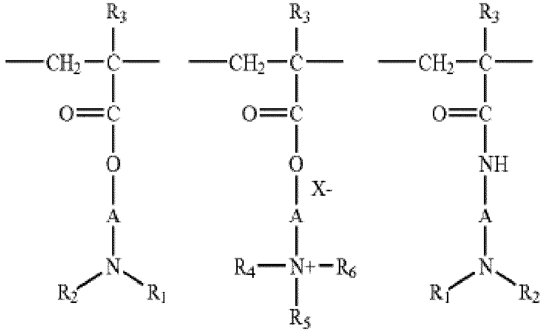 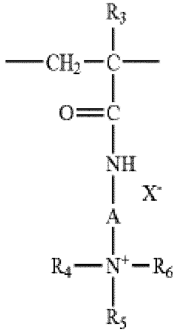 wherein:
   R₁ and R₂, which may be identical or different, are chosen from hydrogen and alkyl groups comprising from 1 to 6 carbon atoms, for instance, methyl and ethyl groups;
   R₃, which may be identical or different, is chosen from hydrogen and CH₃;
   the symbols A, which may be identical or different, are chosen from linear or branched alkyl groups comprising from 1 to 6 carbon atoms, for example, from 2 to 3 carbon atoms and hydroxyalkyl groups comprising from 1 to 4 carbon atoms;
   R₄, R₅, and R₆, which may be identical or different, are chosen from alkyl groups comprising from 1 to 18 carbon atoms and benzyl groups, and in at least one embodiment, alkyl groups comprising from 1 to 6 carbon atoms; and
   X is an anion derived from an inorganic or organic acid, such as methosulphate anions and halides, for instance chloride and bromide.

   The copolymers of family (1) may also comprise at least one unit derived from comonomers which may be chosen from acrylamides, methacrylamides, diacetone acrylamides, acrylamides and methacrylamides substituted on the nitrogen atom with (C₁-C₄) lower alkyl groups, groups derived from acrylic or methacrylic acids and esters thereof, vinyllactams such as vinylpyrrolidone and vinylcaprolactam, and vinyl esters.
   Examples of copolymers of family (1) include, but are not limited to:
   copolymers of acrylamide and of dimethylaminoethyl methacrylate quaternized with dimethyl sulphate or with a dimethyl halide,
   copolymers of acrylamide and of methacryloyloxyethyltrimethylammonium chloride described, for example, in European Patent Application No. 0 080 976,
   copolymers of acrylamide and of methacryloyloxyethyltrimethylammonium methosulphate, quaternized or nonquaternized vinylpyrrolidone/dialkylaminoalkyl acrylate or methacrylate copolymers, described, for example, in French Patent Nos. 2 077 143 and 2 393 573,
   dimethylaminoethyl methacrylate/vinylcaprolactam/vinylpyrrolidone terpolymers, vinylpyrrolidone/methacrylamidopropyldimethylamine copolymers, quaternized vinylpyrrolidone/dimethylaminopropylmethacrylamide copolymers, and
   crosslinked methacryloyloxy(C₁-C₄)alkyltri(C₁-C₄)alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with a compound containing olefinic unsaturation, for example, methylenebisacrylamide.
(2) Cationic cellulose derivatives such as cellulose ether derivatives comprising quaternary ammonium groups described, for example, in French Patent No. 1 492 597, such as the polymers sold under the names "JR" (JR 400, JR 125, JR 30M) or "LR" (LR 400, LR 30M) by the company Union Carbide Corporation. These polymers are also defined in the CTFA dictionary as quaternary ammoniums of hydroxyethylcellulose that have reacted with an epoxide substituted with a trimethylammonium group.
(3) Cationic cellulose derivatives such as cellulose copolymers and cellulose derivatives grafted with a water-soluble monomer of quaternary ammonium, and described, for example, in U.S. Pat. No. 4,131,576, such as hydroxyalkylcelluloses, for instance, hydroxymethyl-, hydroxyethyl-, and hydroxypropylcelluloses grafted, for example, with a salt chosen from methacryloylethyltrimethylammonium, methacrylamidopropyltrimethylammonium, and dimethyldiallylammonium salts.
   Commercial products corresponding to these polymers include, for example, the products sold under the name "Celquat® L 200" and "Celquat® H 100" by the company National Starch.
(4) Non-cellulose-based cationic polysaccharides described in U.S. Pat. Nos. 3,589,578 and 4,031,307, such as guar gums comprising cationic trialkylammonium groups. Guar gums modified with a salt, for example the chloride, of 2,3-epoxypropyltrimethylammonium may also be used.
   Such products are sold, for instance, under the trade names JAGUAR@ C13 S, JAGUAR@ C15, JAGUAR@ C17, and JAGUAR@ C162 by the company MEYHALL.
(5) Polymers comprising piperazinyl units and divalent alkylene or hydroxyalkylene groups comprising straight or branched chains, optionally interrupted with at least one entity chosen from oxygen, sulphur, nitrogen, aromatic rings, and heterocyclic rings, and also the oxidation and/or quaternization products of these polymers. Such polymers are described, for example, in French Patent Nos. 2 162 025 and 2 280 361.
(6) Water-soluble polyamino amides prepared, for example, by polycondensation of an acidic compound with a polyamine; these polyamino amides possibly being crosslinked with an entity chosen from epihalohydrins; diepoxides; dianhydrides; unsaturated dianhydrides; bisunsaturated derivatives; bishalohydrins; bisazetidiniums; bishaloacyidiamines; bisalkyl halides; oligomers resulting from the reaction of a difunctional compound which is reactive with an entity chosen from bishalohydrins, bisazetidiniums, bishaloacyldiamines, bisalkyl halides, epihalohydrins, diepoxides, and bisunsaturated derivatives; the crosslinking agent being used in an amount ranging from 0.025 to 0.35 mol per amine group of the polyamino amide; these polyamino amides optionally being alkylated or, if they comprise at least one tertiary amine function, they may be quaternized. Such polymers are described, for example, in French Patent Nos. 2 252 840 and 2 368 508.
(7) Polyamino amide derivatives resulting from the condensation of polyalkylene polyamines with polycarboxylic acids, followed by alkylation with difunctional agents, for example, adipic acid/dialkylaminohydroxyalkyldialkylenetriamine polymers in which the alkyl group comprises from 1 to 4 carbon atoms, such as methyl, ethyl, and propyl groups, and the alkylene group comprises from 1 to 4 carbon atoms, such as an ethylene group. Such polymers are described, for instance, in French Patent No. 1 583 363. In at least one embodiment, these derivatives may be chosen from adipic acid/dimethylaminohydroxypropyldiethylenetriamine polymers.
(8) Polymers obtained by reaction of a polyalkylene polyamine comprising two primary amine groups and at least one secondary amine group, with a dicarboxylic acid chosen from diglycolic acid and saturated aliphatic dicarboxylic acids comprising from 3 to 8 carbon atoms. The molar ratio of the polyalkylene polyamine to the dicarboxylic acid may range from 0.8:1 to 1.4:1; the polyamino amide resulting therefrom being reacted with epichlorohydrin in a molar ratio of epichlorohydrin relative to the secondary amine group of the polyamino amide ranging from 0.5:1 to 1.8:1. Such polymers are described, for example, in U.S. Pat. Nos. 3,227,615 and 2,961,347.
(9) Cyclopolymers of alkyldiallylamine and cyclopolymers of dialkyldiallyl-ammonium, such as homopolymers and copolymers comprising, as the main constituent of the chain, at least one unit chosen from units of formulas (Va) and (Vb): 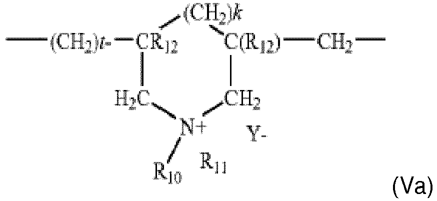 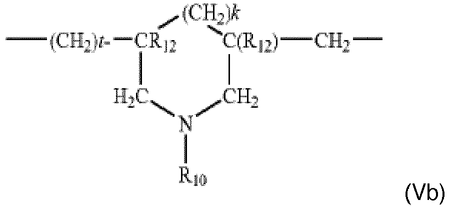 wherein:
   k and t, which may be identical or different, are equal to 0 or 1, the sum k+t being equal to 1; R₁₂ is chosen from hydrogen and methyl groups;
   R₁₀ and R₁₁, which may be identical or different, are chosen from alkyl groups comprising from 1 to 6 carbon atoms, hydroxyalkyl groups in which the alkyl group comprises, for example, from 1 to 5 carbon atoms, and lower (C₁-C₄)amidoalkyl groups, or R₁₀ and R₁₁ may form, together with the nitrogen atom to which they are attached, heterocyclic groups such as piperidinyl and morpholinyl; and
   Y' is an anion such as bromide, chloride, acetate, borate, citrate, tartrate, bisulphate, bisulphite, sulphate, and phosphate. These polymers are described, for example, in French Patent No. 2 080 759 and in its Certificate of Addition 2 190 406.

In one embodiment, R₁₀ and R₁₁, which may be identical or different, are chosen from alkyl groups comprising from 1 to 4 carbon atoms.

Examples of such polymers include, but are not limited to, the dimethyidiallylammonium chloride homopolymer sold under the name "MERQUAT® 100" by the company CALGON (and its homologues of low weight-average molecular mass) and the copolymers of diallyldimethylammonium chloride and of acrylamide sold under the name "MERQUAT® 550".

Quaternary diammonium polymers comprising at least one repeating unit of formula (VI): 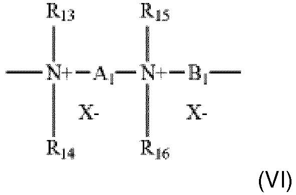 wherein:
R₁₃, R₁₄, R₁₅, and R₁₆, which may be identical or different, are chosen from aliphatic, alicyclic, and arylaliphatic groups comprising from 1 to 20 carbon atoms and lower hydroxyalkyl aliphatic groups, or alternatively R₁₃, R₁₄, R₁₅, and R₁₆ may form, together or separately, with the nitrogen atoms to which they are attached, heterocycles optionally comprising a second heteroatom other than nitrogen, or alternatively R₁₃, R₁₄, R₁₅, and R₁₆, which may be identical or different, are chosen from linear or branched C₁-C₆ alkyl groups substituted with at least one group chosen from nitrile groups, ester groups, acyl groups, amide groups, -CO-O-R₁₇-E groups, and -CO-NH-R₁₇-E groups, wherein R₁₇ is an alkylene group and E is a quaternary ammonium group;
A₁ and B₁, which may be identical or different, are chosen from polymethylene groups comprising from 2 to 20 carbon atoms, which may be linear or branched, saturated or unsaturated, and which may comprise, linked or intercalated in the main chain, at least one entity chosen from aromatic rings, oxygen, sulphur, sulphoxide groups, sulphone groups, disulphide groups, amino groups, alkylamino groups, hydroxyl groups, quaternary ammonium groups, ureido groups, amide groups, and ester groups, and
X⁻ is an anion derived from an inorganic or organic acid;
A₁, R₁₃, and R₁₅ may form, together with the two nitrogen atoms to which they are attached, a piperazine ring;
if A₁ is chosen from linear or branched, saturated or unsaturated alkylene or hydroxyalkylene groups, B₁ may be chosen from:

   -(CH₂)ₙ--CO-E'-OC-(CH₂)ₙ-
wherein E' is chosen from:
   a) glycol residues of formula -O-Z-O-, wherein Z is chosen from linear or branched hydrocarbon-based groups and groups of the following formulas:

      -(CH₂-CH₂-O)ₓ-CH₂-CH₂-

      -[CH₂-CH(CH₃)-O]_{y}-CH₂-CH(CH₃)-

      wherein x and y, which may be identical or different, are chosen from integers ranging from 1 to 4, which represent a defined and unique degree of polymerization, and numbers ranging from 1 to 4, which represent an average degree of polymerization;
   b) bis-secondary diamine residue such as piperazine derivatives;
   c) bis-primary diamine residues of formula -NH-Y-NH-, wherein Y is chosen from linear or branched hydrocarbon-based groups and the divalent group -CH₂-CH₂-S-S-CH₂-CH₂-_{;} and
   d) ureylene groups of formula -NH-CO-NH-.

In at least one embodiment, X⁻ is an anion such as chloride or bromide.

Polymers of this type are described, for example, in French Patent Nos. 2 320 330; 2 270 846; 2 316 271; 2 336 434; and 2 413 907 and U.S. Pat. Nos. 2,273,780; 2,375,853; 2,388,614; 2,454,547; 3,206,462; 2,261,002; 2,271,378; 3,874,870; 4,001,432; 3,929,990; 3,966,904; 4,005,193; 4,025,617; 4,025,627; 4,025,653; 4,026,945; and 4,027,020.

Non-limiting examples of such polymers include those comprising at least one repeating unit of formula (VII): 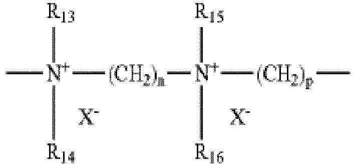 wherein R₁₃, R₁₄, R₁₅, and R₁₆, which may be identical or different, are chosen from alkyl and hydroxyalkyl groups comprising from 1 to 4 carbon atoms, n and p, which may be identical or different, are integers ranging from 2 to 20, and X⁻ is an anion derived from an inorganic or organic acid.
(11) Polyquaternary ammonium polymers comprising units of formula (VIII): 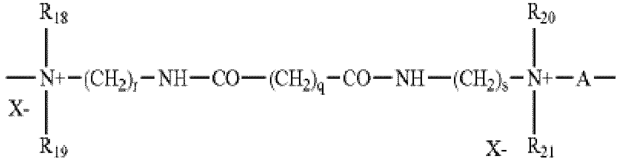 wherein:
   R₁₈, R₁₉, R₂₀, and R₂₁, which may be identical or different, are chosen from hydrogen, methyl groups, ethyl groups, propyl groups, 3-hydroxyethyl groups, 3-hydroxypropyl groups, - CH₂CH₂(OCH₂CH₂)ₚOH groups, wherein p is chosen from integers ranging from 0 to 6, with the proviso that R₁₈, R₁₉, R₂₀, and R₂₁ are not simultaneously hydrogen,
   r and s, which may be identical or different, are chosen from integers ranging from 1 to 6,
   q is chosen from integers ranging from 0 to 34,
   X⁻ is an anion such as a halide, and
   A is chosen from radicals of dihalides and -CH₂-CH₂-O-CH₂-CH₂-.

   Such compounds are described, for instance, in European Patent Application No. 0 122 324.
(12) Quaternary polymers of vinylpyrrolidone and of vinylimidazole.

Other examples of suitable cationic polymers include, but are not limited to, cationic proteins and cationic protein hydrolysates, polyalkyleneimines, such as polyethyleneimines, polymers comprising units chosen from vinylpyridine and vinylpyridinium units, condensates of polyamines and of epichlorohydrin, quaternary polyureylenes, and chitin derivatives.

According to one embodiment of the present invention, the at least one cationic polymer is chosen from cellulose ether derivatives comprising quaternary ammonium groups, such as the products sold under the name "JR 400" by the company UNION CARBIDE CORPORATION, cationic cyclopolymers, for instance, the homo-polymers and copolymers of dimethyldiallylammonium chloride sold under the names MERQUAT® 100, MERQUAT® 550, and MERQUAT® S by the company CALGON, guar gums modified with a 2,3-epoxypropyltrimethylammonium salt, and quaternary polymers of vinylpyrrolidone and of vinylimidazole.

The at least one cationic polymer may be present in the composition of the present invention in an amount of at least 0.01% by weight relative to the total weight of the composition, for example, from 0.01 to 10% by weight, from 0.1 to 5% by weight, or from 0.1 to 3% by weight, relative to the total weight of the composition.

The composition used for the present invention contains neither a reducing agent nor a source of carbonate ions of the formula: 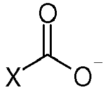 wherein
X is a group selected from the group consisting of O⁻, OH, NH₂, O-OH, and O-COO⁻.

The pH of the composition may range from 6 to 11, preferably between 6.0 and 9.0, and more preferably between 6.0 to 8.0. Since the pH of the composition is not relatively high or low, damage to the keratin fibers by the composition can be reduced.

In order to adjust the pH, an acidic or alkali agent(s) other than sources of ions of the invention may be used alone or in combination. The amount of the acidic or alkali agent(s) is not limited, but may be from 0.1 to 5% by weight relative to the total weight of the composition. As the acidic agents, mention may be made of any inorganic or organic acids which are commonly used in cosmetic products such as citric acid, lactic acid, phosphoric acid or hydrochloric acid (HCI). HCI is preferable. As the alkali agents, mention may be made of any inorganic or organic basic agents which are commonly used in cosmetic products such as ammonia; alcanolamines such as mono-, di- and tri-ethanolamine, isopropanolamine; sodium and potassium hydroxides; urea, guanidine and their derivatives; basic amino acids such as lysine or arginine; and diamines such as those described in the structure below: 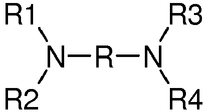 wherein R denotes an alkylene such as propylene optionally substituted by a hydroxyl or a C₁-C₄ alkyl radical, and R₁, R₂, R₃ and R₄ independently denote a hydrogen atom, an alkyl radical or a C₁-C₄ hydroxyalkyl radical, which may be exemplified by 1,3-propanediamine and derivatives thereof. Arginine and monoethanolamine are preferred.

The composition used for the present invention may also comprise one or more additional cosmetic agent(s). The amount of the additional cosmetic agent(s) is not limited, but may be from 0.1 to 10% by weight relative to the total weight of the composition. The cosmetic agent(s) may be selected from the group consisting of volatile or non volatile, linear or cyclic, amine-type or not, silicones, anionic, non ionic or amphoteric polymers, peptides and derivatives thereof, protein hydrolyzates, synthetic or natural waxes, and especially fatty alcohols, swelling agents and penetrating agents, as well as other active compounds, such as anionic, cationic, non ionic, amphoteric or zwitterionic surfactants, agents for combating hair loss, anti-dandruff agents, associative-type or not, natural or synthetic thickeners, suspending agents, sequestering agents, opacifying agents, dyes, sunscreen agents, fillers, vitamins or provitamins, mineral, vegetable or synthetic oils, as well as fragrances, preserving agents, stabilizers, and mixtures thereof.

The vehicle for the composition used for the present invention is preferably an aqueous medium consisting of water and may advantageously contain one or several cosmetically acceptable organic solvents, which particularly include alcohols, such as ethyl alcohol, isopropyl alcohol, benzyl alcohol and phenylethyl alcohol, or polyols or polyol ethers, such as ethylene glycol monomethyl, monoethyl and monobutyl ethers, propylene glycol or ethers thereof, such as propylene glycol monomethylether, butylene glycol, dipropylene glycol as well as diethylene glycol alkyl ethers, such as diethylene glycol monoethylether or monobutylether. The water may be present in a concentration of from 10 to 90% by weight relative to the total weight of the composition. The organic solvent(s) may then be present in a concentration of from 0.1 to 20% by weight, and preferably from 1 to 10% by weight relative to the total weight of the composition.

The composition used in the present invention may exist in any form such as a lotion, a gel, thickened or not, or a cream.

### (Keratin Fiber Treatment Process)

The process for treating keratin fibers according to the present invention can be performed by applying onto the keratin fibers a composition comprising at least one cationic polymer, as described above;
then placing the keratin fibers in an occlusive space; and
then heating the keratin fibers,

According to the present invention relating to the treatment process for keratin fibers, keratin fibers such as hair are subjected to a specific heating process which is performed in an occlusive space.

The heating process can be performed by any heating means which can be freely controlled to realize the temperature desired for the process.

The heating process may preferably be performed by using a special heating device or devices that can form an occlusive space to restrict the evaporation of evaporable components such as water in the above-described composition from keratin fibers and keep a predetermined temperature in the heating device throughout the process.

If the evaporable components such as water in the above-described composition evaporate from the keratin fibers, most of the heat energy applied to the keratin fibers will be consumed by the evaporation, and therefore the temperature of the keratin fibers cannot increase up to the predetermined temperature until all evaporable components in the composition evaporate.

The above heating device may comprise a heat energy source being either in contact with keratin fibers or apart from keratin fibers, and at least one means to form an occlusive space surrounding the keratin fibers.

The heat energy source is used to heat keratin fibers. The heat energy source may be at least one heater providing at least one selected from the group consisting of hot air, hot steam, high frequency induction heating, microwave heating, infrared ray irradiation, laser, and flash lamp irradiation.

The occlusive space may be formed by at least one coating means. A plurality of coating means may be used. The coating means may be rigid or flexible.

The coating means may comprise at least one member selected from the group consisting of a film and a sheet. The material of the film or the sheet is not limited. For example, the film or the sheet may comprise a thermoplastic or thermosetting resin, a paper, a textile, a bonnet, a metal foil such as aluminum foil, and the like.

For example, the film or sheet may be set on a heating rod, a heating bar or a heating plate which is covered by keratin fibers.

According to the present invention, the coating means may comprise the heat energy source. Therefore, for example, the film or sheet which includes a heater may be set on a rod, a bar, or a plate which is covered by keratin fibers.

The occlusive conditions restrict the evaporation of evaporable components such as water in the above-described composition applied to keratin fibers, and therefore the temperature of the keratin fibers can be increased higher than that obtainable by a conventional heating process or device for the keratin fibers in open conditions. Furthermore, the keratin fibers can be heated effectively, and the keratin fibers can be heated evenly.

According to one variant of the present invention, the occlusive space may comprise apertures, the surface area of which is less than 5%, preferably less than 3% and more particularly less than 0.5% of the total surface area of the coating means. According to this variant, the total surface area of the coating means comprises the surface area of, when it is present, an opening means for the coating means.

The apertures may be passages, holes or orifices, which may allow an exchange of air between the occlusive space and the exterior thereof, especially when the reaction such as forming vapor inside the occlusive space is too great. On the other hand, a person skilled in the art could form the apertures such that the diffusion of heat in the occlusive space is not impaired.

The keratin fibers are heated at 45ºC to 250ºC, preferably 60ºC to 200ºC, more preferably 60ºC to 150ºC, more preferably 60ºC to 90ºC, during the step of heating the keratin fibers.

The heating process may be performed for an appropriate time which is required to treat keratin fibers. The time length for the heating process is not limited, but it may be from 1 minute to 2 hours, preferably 1 minute to 1 hour, and more preferably 1 minute to 30 minutes. For example, the time for heating may be from 5 to 20 minutes, preferably 10 to 15 minutes.

The keratin fibers may be rinsed after the step of applying the composition onto the keratin fibers and/or after the step of heating the keratin fibers.

### (Permanent Deformation Process for Keratin Fiber)

According to the present invention relating to the treatment process for keratin fibers, the keratin fiber may be subjected to mechanical tension which is typically used for permanent deformation.

The permanent deformation process for keratin fibers when mechanical tension is applied to keratin fibers may be performed as follows.

First, keratin fibers are subjected to mechanical tension for deformation. The mechanical tension can be applied to the keratin fibers by any means to deform the keratin fibers to an intended shape. For example, the mechanical tension may be provided by at least one reshaping means selected from the group consisting of a curler, a roller, a clip, a plate and an iron. The reshaping means may comprise at least one heater as described above. If the keratin fibers are rolled around a curler, this rolling-up may be performed on the entire length of the keratin fibers or, for example, on half the length of the keratin fibers. Depending on, for example, the desired hairstyle shape and amount of curls, the rolling-up may be performed with more or less thick locks.

Next, the above-described composition is applied to the keratin fibers. The application of the composition may be performed by any means, such as a brush and a comb. The keratin fibers to which the mechanical tension has been applied should be treated with the composition. It may be possible that the keratin fibers are left as they are for a certain amount of time, if necessary.

Lastly, the above-described heating process is performed. The heat energy is applied to the keratin fibers under occlusive conditions as described above.

This process for permanent deformation of keratin fibers may be performed without any step of oxidizing the keratin fibers. Therefore, the time required for the process according to the present invention can be shorter than that for a conventional process which needs an oxidizing step. Furthermore, damage to the keratin fibers by the oxidizing step can be avoided.

The keratin fibers may be rinsed after the step of applying the composition onto the keratin fibers and/or after the step of heating the keratin fibers.

One embodiment of the cosmetic treatment process according to the present invention may be a process for reshaping or permanent deforming keratin fibers, in particular hair, comprising:
a) a step of placing the keratin fibers under mechanical tension by rolling them up on at least one reshaping or mechanically tensioning means so as to form curls;
b) a step of applying the above-described composition to the keratin fibers;
c) an optional step of rinsing the keratin fibers,
d) a step of placing at least one coating means on the reshaping or mechanically tensioning means or vice versa to form one or more occlusive spaces; and
e) a step of heating the keratin fibers at a temperature of between 45 ± 2 or 3°C and 250 ± 2 or 3°C for 1 minute to 2 hours.

In this process, the temperature can be set, adjusted and regulated by using one or more heating means, and may be measured with a thermo-measurement probe such as Digital Surface Sensor Module, reference MT-144, sold by Sakaguchi E.H VOC Corp (Japan), set on the keratin fibers. Normally, the probe is set on a single keratin fiber. However, it is advantageous that the probe is set on the part of the keratin fibers which directly contacts with the occlusive space, and more preferably, the probe is set on the part of the keratin fibers which directly contacts with the occlusive space and forms the curl end of the keratin fibers, if a curler is used.

Preferably, the temperature is measured at atmospheric pressure of 101325 Pa.

According to the present invention, the temperature of the keratin fibers may be constant with a fluctuation of ± 2 or 3°C over the head, if the keratin fibers are hair, of an individual, and the probe may be set on any type of keratin fibers.

If the keratin fibers are hair, according to the present invention, the constant temperature with a fluctuation of ± 2 or 3°C can be obtained for any type of hair, and the temperature of the hair can be controlled to be constant ± 2 or 3°C during the heating of the hair at a certain temperature. Thus, the hair style becomes uniform and homogeneous for the entirety of the hair, and a more excellent hair style can be finally obtained.

Advantageously, the coating means may comprise one or more thermal insulating materials, and more advantageously, the coating means may consist of the material(s).

The term "thermal insulating material" means any material which has an electric conductivity of 0 to 1 W/m°C (PVC: 0.17 W/m°C).

Preferably, the heating means may be adjusted such that the temperature measured on the keratin fibers is 50°C or more, more preferably 55°C to less than 150°C, and further more preferably less than 100°C. It is preferable that the heating is performed by heating via electrical resistance.

Advantageously, the coating means is impermeable with regard to the composition used in the step b).

In the above embodiment, at least one of the reshaping or mechanical tensioning means and at least one of the covering means may include a heater.

In the above embodiment, "occlusive space" means that when the coating means is placed on the reshaping or mechanical tensioning means, or vice versa, they together form a closed structure in which heat can diffuse, but heat cannot diffuse out of or is difficult to diffuse out of the closed structure. It is preferable that the coating means and the reshaping or mechanical tensioning means can form the occlusive space when they are set on the head, if the keratin fibers are hair.

The occlusive space may form a condensation cage in which water and a component or components in the composition used in the step b) may evaporate from the keratin fibers, adhere to the wall of the coating means, and drop onto the keratin fibers. This cycle may be repeated during the heating of the keratin fibers. Thus, the keratin fibers can be always kept wet, and drying and deteriorating of the keratin fibers will be prevented.

The formation of occlusive space is an important characteristic of the present invention, because the keratin fibers in the occlusive space can be kept wet and the temperature of the keratin fibers can be constant.

Preferably, the process of the present invention may comprise an additional step of tightening the coating means on the head of an individual, if the keratin fibers are hair, by an elastic cord, an extensible band, or a stretch.

According to the process of the present invention, because of the occlusive space in which the composition can be continuously condensed on the keratin fibers, the amount of a cosmetic component or components in the composition is advantageously reduced as compared to the processes in the prior art. The amount of the cosmetic component(s) may be 0.3 to 3wt% of the composition.

In a preferred embodiment, a coating means may be placed on each hair curler as the reshaping or mechanically tensioning means, if the keratin fibers are hair. In other words, each of the hair curlers, if two or more hair curlers are used, may be covered individually by a coating means. It is advantageous to cover each hair curler because leaking to the scalp of the composition which has been applied onto keratin fibers in the step b) can be prevented.

In another preferred embodiment, a coating means may cover all hair curlers, if two or more hair curlers are used. In other words, the coating means may cover the entirety of the head if the keratin fibers are hair.

Advantageously, the occlusive space formed in the step d) may be maintained during the step e). In other words, the coating means may be removed only after the step e) or after the stop of the heating in the step e).

If necessary, the composition may be applied to keratin fibers before applying mechanical tension to the keratin fibers. It may be possible that the keratin fibers are left as they are for a certain amount of time, if necessary, before and/or after applying mechanical tension to the keratin fibers, before and/or after applying the above-described composition to the keratin fibers, and before and/or after heating the keratin fibers.

After the above step e), if necessary, the keratin fibers may be fixed by oxidation after being taken out from the coating means.

### (Products)

The present application also disclose a composition for treating keratin fibers to be heated in an occlusive space, comprising at least one cationic polymer,
wherein
the composition contains neither a reducing agent nor a source of carbonate ions of the formula: 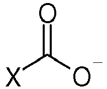 wherein
X is a group selected from the group consisting of O⁻, OH, NH₂, O-OH, and O-COO⁻.

This composition may not need to be used in combination with an oxidizing agent which is used in a conventional permanent deformation of keratin fibers. Therefore, if keratin fibers should be permanently deformed, the composition may be used in one step, whereas two steps (reducing step and oxidizing step) are necessary in the conventional permanent deformation of keratin fibers.

This composition may have the same technical features as those of the composition described above.

The present application also discloses a kit for treating keratin fibers, comprising:
a device comprising
   at least one reshaping means to provide the keratin fibers with mechanical tension,
   at least one coating means to form an occlusive space, and
   at least one heater to heat the keratin fibers in the occlusive space;
      and
a composition comprising at least one cationic polymer wherein the composition contains neither a reducing agent nor a source of carbonate ions of the formula: 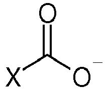 wherein
   X is a group selected from the group consisting of O⁻, OH, NH₂, O-OH, and O-COO⁻.

The coating means and the heater, as well as the composition in the kit, may be the same as those described above.

### EXAMPLES

The present invention will be described in more detail by way of examples, which however should not be construed as limiting the scope of the present invention.

### Composition 1

A hair treatment composition (referred to as "Composition 1") having the following composition shown in Table 1 was prepared (active ingredients in wt %).

**Table 1**

| | |
|---|---|
| Homopolymers of diallyldimethylammonium chloride at 40% in water (Merquat 100) | 1.5 |
| Hydroxyethylcellulose (MW 720,000) | 0.7 |
| Decylpolyglucoside | 9 |
| Preservatives | qs |
| pH adjuster | qsp pH 7±0.2 |
| Water | qsp 100 |

### Example 1

Composition 1 was applied for 15 minutes on a 1g natural Japanese hair swatch previously wrapped on a 1.7 cm heating perm-roller. Then, the perm-roller was covered by a plastic film and plugged into a Digital Perm Machine (Oohiro, model ODIS-2). After a heating process at 90°C for 15 minutes, the hair was rinsed, removed from the perm-roller and dried.

The hair was soft, smooth and slippery or silky. These cosmetic properties were still observed after several shampoos.

### Comparative Example 1

Composition 1 was applied for 15 minutes on a 1g natural Japanese hair swatch which was the same as that used in Example 1. After leaving the hair without heating for 15 minutes, the hair was rinsed and dried.

No specific change was observed on hair after the treatment.

### Composition 2

A hair treatment composition (referred to as "Composition 2") having the following composition shown in Table 2 was prepared (active ingredients in wt %).

**Table 2**

| | |
|---|---|
| Hexadimethrine chloride at 60% in water (Mexomer PO) | 3 |
| Hydroxyethylcellulose (MW 720,000) | 0.7 |
| Decylpolyglucoside | 9 |
| Preservatives | qs |
| pH adjuster | qsp pH 7±0.2 |
| Water | qsp 100 |

### Example 2

Composition 2 was applied for 15 minutes on a 1g natural Japanese hair swatch previously wrapped on a 1.7 cm heating perm-roller. Then, the perm-roller was covered by a plastic film and plugged into a Digital Perm Machine (Oohiro, model ODIS-2). After a heating process at 90°C for 15 minutes, the hair was rinsed, removed from the perm-roller and dried.

The hair was soft and easy to comb. These cosmetic properties were still observed after several shampoos.

### Comparative Example 2

Composition 2 was applied for 15 minutes on a 1g natural Japanese hair swatch which was the same as that used in Example 2. After leaving the hair without heating for 15 minutes, the hair was rinsed and dried.

No specific change was observed on the hair after the treatment.

## Claims

1. A permanent deformation process for keratin fibers comprising the steps of:
providing the keratin fibers with mechanical tension;
applying onto the keratin fibers a composition comprising at least one cationic polymer;
then placing the keratin fibers in an occlusive space to restrict the evaporation of evaporable components in the composition and keep the keratin fibers wet;
and then heating the keratin fibers at a temperature ranging from 45°C to 250°C while keeping the keratin fibers in the wet state,
wherein the composition contains neither a reducing agent nor a source of carbonate ions of the formula: 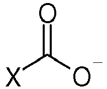 wherein
X is a group selected from the group consisting of O⁻, OH, NH₂, O-OH, and O-COO⁻.

2. The process according to Claim 1, further comprising the step of rinsing the keratin fibers after the step of applying the composition onto the keratin fibers and/or after the step of heating the keratin fibers.

3. The process according to Claim 1 or 2, wherein the step of providing the keratin fibers with mechanical tension is performed after the step of applying onto the keratin fibers a composition comprising at least one cationic polymer.

4. The process according to any one of Claims 1 to 3, wherein the occlusive space is formed by at least one coating means.

5. The process according to Claim 4, wherein the coating means is rigid or flexible.

6. The process according to Claim 4 or 5, wherein the coating means comprises at least one member selected from the group consisting of a film and a sheet.

7. The process according to any one of Claims 1 to 6, wherein the keratin fibers are heated at 60ºC to 200ºC during the step of heating the keratin fibers.

8. The process according to any one of Claims 1 to 7, wherein the keratin fibers are heated by at least one heater providing at least one selected from the group consisting of hot air, hot steam, high frequency induction heating, microwave heating, infra-red ray irradiation, laser, and flash lamp irradiation.

9. The process according to Claim 8, wherein the coating means comprises the heater.

10. The process according to any one of Claims 1 to 9, wherein the cationic polymer has a molecular weight of more than 5000.

11. The process according to any one of Claims 1 to 10, wherein the cationic polymer has no siloxane moiety.

12. The process according to any one of Claims 1 to 11, wherein the cationic polymer comprises at least one group chosen from primary, secondary, tertiary, and/or quaternary amine groups which may either be part of the main polymer chain, or may be carried by a side substituent directly connected to the main chain.

13. The process according to any one of Claims 1 to 12, wherein the cationic polymer is selected from the group consisting of:
(1) homopolymers and copolymers derived from acrylic or methacrylic esters and amides;
(2) cationic polysaccharides;
(3) polyaminoamides and derivatives thereof;
(4) cyclopolymers of alkyldiallylamine or dialkyldiallylammonium;
(5) quaternary diammonium polymers;
(6) quaternary polyammonium polymers; and
(7) quaternary polymers of vinylpyrrolidone or vinylimidazole.

## Patentansprüche

1. Verfahren zum dauerhaften Verformen für Keratinfasern, das die Schritte umfasst:
Anlegen einer mechanischen Spannung an die Keratinfasern;
Auftragen einer Zusammensetzung, die zumindest ein kationisches Polymer umfasst, auf die Kartinfasern;
danach Platzieren der Keratinfasern in einem verschlossenen Raum, um die Verdampfung von verdampfbaren Komponenten in der Zusammensetzung zu begrenzen und die Keratinfasern nass zu halten;
und danach Erhitzen der Keratinfasern bei einer Temperatur im Bereich von 45 °C bis 250 °C, während die Keratinfasern im nassen Zustand gehalten werden,
wobei die Zusammensetzung weder ein Reduktionsmittel noch eine Quelle von Carbonationen der Formel enthält: 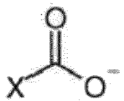 wobei
X eine Gruppe ist, die aus der Gruppe ausgewählt ist, bestehend aus O⁻, OH, NH₂, O-OH und O-COO⁻.

2. Verfahren nach Anspruch 1, das ferner den Schritt des Spülens der Keratinfasern nach dem Schritt des Auftragens der Zusammensetzung auf die Keratinfasern und/oder nach dem Schritt des Erhitzens der Keratinfasern umfasst.

3. Verfahren nach Anspruch 1 oder 2, wobei der Schritt des Anlegens einer mechanischen Spannung an die Keratinfasern nach dem Schritt des Auftragens einer Zusammensetzung, die zumindest ein kationisches Polymer umfasst, auf die Keratinfasern durchgeführt wird.

4. Verfahren nach einem der Ansprüche 1 bis 3, wobei der verschlossene Raum durch zumindest ein Beschichtungsmittel gebildet ist.

5. Verfahren nach Anspruch 4, wobei das Beschichtungsmittel starr oder flexibel ist.

6. Verfahren nach Anspruch 4 oder 5, wobei das Beschichtungsmittel zumindest ein Element umfasst, das aus der Gruppe ausgewählt ist, bestehend aus einem Film und einer Folie.

7. Verfahren nach einem der Ansprüche 1 bis 6, wobei die Keratinfasern während des Schritts des Erhitzens der Keratinfasern bei 60 °C bis 200 °C erhitzt werden.

8. Verfahren nach einem der Ansprüche 1 bis 7, wobei die Keratinfasern durch zumindest ein Heizelement erhitzt werden, das zumindest eines bereitstellt, das aus der Gruppe ausgewählt ist, bestehend aus heißer Luft, heißem Dampf, hochfrequenter Induktionserhitzung, Mikrowellenerhitzung, Infrarotstrahlbestrahlung, Laser- und Blitzlampenbestrah lung.

9. Verfahren nach Anspruch 8, wobei das Beschichtungsmittel das Heizelement umfasst.

10. Verfahren nach einem der Ansprüche 1 bis 9, wobei das kationische Polymer ein Molekulargewicht von mehr als 5000 aufweist.

11. Verfahren nach einem der Ansprüche 1 bis 10, wobei das kationische Polymer keine Siloxaneinheit aufweist.

12. Verfahren nach einem der Ansprüche 1 bis 11, wobei das kationische Polymer zumindest eine Gruppe umfasst, die aus primären, sekundären, tertiären und/oder quaternären Amingruppen ausgewählt ist, die entweder ein Teil der Hauptpolymerkette sein kann oder von einem Seitensubstituenten getragen werden kann, der direkt mit der Hauptkette verbunden ist.

13. Verfahren nach einem der Ansprüche 1 bis 12, wobei das kationische Polymer aus der Gruppe ausgewählt ist, bestehend aus:
(1) Homopolymeren und Copolymeren, die von Acryl- oder Methacrylestern und -amiden abgeleitet sind;
(2) kationischen Polysacchariden;
(3) Polyaminoamiden und Derivaten davon;
(4) Cyclopolymeren von Alkyldiallylamin oder Dialkyldiallylammonium;
(5) quaternären Diammoniumpolymeren;
(6) quaternären Polyammoniumpolymeren; und
(7) quaternären Polymeren von Vinylpyrrolidon oder Vinylimidazol.

## Revendications

1. Procédé de déformation permanente de fibres de kératine, comprenant les étapes de :
soumission des fibres de kératine à une tension mécanique ;
application sur les fibres de kératine d'une composition comprenant au moins un polymère cationique ;
puis placement des fibres de kératine dans un espace occlusif pour limiter l'évaporation de composants évaporables dans la composition et maintenir les fibres de kératine humides ;
puis chauffage des fibres de kératine à une température située dans la plage allant de 45°C à 250°C tout en maintenant les fibres de kératine à l'état humide,
dans lequel la composition ne contient ni agent réducteur ni source d'ions carbonate de formule : 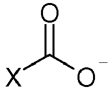
dans laquelle X est un groupe choisi dans l'ensemble constitué par O⁻, OH, NH₂, O-OH, et O-COO⁻.

2. Procédé selon la revendication 1, comprenant en outre l'étape de rinçage des fibres de kératine après l'étape d'application de la composition sur les fibres de kératine et/ou après l'étape de chauffage des fibres de kératine.

3. Procédé selon la revendication 1 ou 2, dans lequel l'étape de soumission des fibres de kératine à une tension mécanique est effectuée après l'étape d'application sur les fibres de kératine d'une composition comprenant au moins un polymère cationique.

4. Procédé selon l'une quelconque des revendications 1 à 3, dans lequel l'espace occlusif est formé par au moins un moyen de revêtement.

5. Procédé selon la revendication 4, dans lequel le moyen de revêtement est rigide ou flexible.

6. Procédé selon la revendication 4 ou 5, dans lequel le moyen de revêtement comprend au moins un membre choisi dans l'ensemble constitué par un film et une feuille.

7. Procédé selon l'une quelconque des revendications 1 à 6, dans lequel les fibres de kératine sont chauffées à une température de 60°C à 200°C durant l'étape de chauffage des fibres de kératine.

8. Procédé selon l'une quelconque des revendications 1 à 7, dans lequel les fibres de kératine sont chauffées par au moins un dispositif chauffant fournissant au moins l'un choisi dans l'ensemble constitué par de l'air chaud, de la vapeur chaude, un chauffage par induction haute fréquence, un chauffage par micro-ondes, une irradiation de rayons infrarouges, un laser, et une irradiation par une lampe flash.

9. Procédé selon la revendication 8, dans lequel le moyen de revêtement comprend le dispositif chauffant.

10. Procédé selon l'une quelconque des revendications 1 à 9, dans lequel le polymère cationique a une masse moléculaire supérieure à 5000.

11. Procédé selon l'une quelconque des revendications 1 à 10, dans lequel le polymère cationique n'a pas de fragment siloxane.

12. Procédé selon l'une quelconque des revendications 1 à 11, dans lequel le polymère cationique comprend au moins un groupe choisi parmi les groupes amino primaire, secondaire, tertiaire et/ou quaternaire qui soit peuvent faire partie de la chaîne polymère principale soit peuvent être portés par un substituant latéral directement connecté à la chaîne principale.

13. Procédé selon l'une quelconque des revendications 1 à 12, dans lequel le polymère cationique est choisi dans l'ensemble constitué par :
(1) les homopolymères et copolymères dérivés d'esters et d'amides d'acide acrylique ou méthacrylique ;
(2) les polysaccharides cationiques ;
(3) les polyaminoamides et leurs dérivés ;
(4) les polymères cycliques d'alkyldiallylamine ou de dialkyldiallylammonium ;
(5) les polymères de diammonium quaternaire ;
(6) les polymères de polyammonium quaternaire ; et
(7) les polymères quaternaires de vinylpyrrolidone ou de vinylimidazole.
